# EUROPÄISCHE PATENTSCHRIFT

(11) **EP 1 072 563 B1**
(45) Veröffentlichungstag und Bekanntmachung des Hinweises auf die Patenterteilung: **03.09.2003**
(21) Anmeldenummer: 00115693.4
(22) Anmeldetag: 21.07.2000
(51) Int. Cl.: C03C 12/00, A61K 6/083

(54) **Glaspulver und dessen Verwendung**
Glass powder and its application
Poudre de verre et son utilisation

(30) Priorität: 29.07.1999 DE 19935707
(43) Veröffentlichungstag der Anmeldung: 31.01.2001
(73) Patentinhaber: Schott Glas, 55122 Mainz (DE); Carl-Zeiss-Stiftung trading as Schott Glas, 55122 Mainz (DE)
(72) Erfinder: Kessler, Susanne, 84030 Ergolding (DE); Paschke, Hartmut, Dr., 84030 Ergolding (DE); Beudt, Hans-Werner, 65187 Wiesbaden (DE); Kiermayer, Susanne, 84056 Rottenburg/Niedereulenbach (DE)

(56) Entgegenhaltungen:
- GB-A- 2 323 366
- US-A- 3 370 966
- US-A- 4 099 977
- US-A- 4 358 549

## Beschreibung

Die Erfindung betrifft ein Glaspulver und dessen Verwendung.

Gläser bzw. daraus hergestellte Glaspulver enthalten oftmals polyvalente Kationen, sei es als absichtlich dem Glasgemenge zugesetzte Komponente, beispielsweise zum Färben eines Glases oder zur Einstellung einer bestimmten Glaseigenschaft, oder sei es als zum Teil nur in Spuren vorliegende Verunreinigung der jeweiligen Gemengekomponenten oder der mit dem Gemenge oder der Schmelze in Berührung stehenden Behältnisse.

Die Oxidationszahl der polyvalenten Kationen wird im wesentlichen durch die Verfahrensführung während der Glasschmelze (reduzierende oder oxidierende Schmelze), durch die Gemenge- bzw. Glaszusammensetzung und/oder durch die Art und Menge der verwendeten Läutermittel eingestellt. Unerwünschte Farbstiche lassen sich beispielsweise während der Läuterung unter Verwendung geeigneter Läutermittel beseitigen.

Aufgrund äußerer Einflüsse, beispielsweise während der Weiterverarbeitung oder Nachbehandlung eines Glases oder eines Glaspulvers, kann sich die Oxidationszahl der polyvalenten Kationen unvorteilhaft ändern.

Insbesondere bei Gläsern oder Glaspulvern, bei denen die Einhaltung einer bestimmten Oxidationszahl auch nach einer Weiterverarbeitung wesentlich ist, z. B. die Einhaltung einer bestimmten Färbung oder einer bestimmten Glaseigenschaft, wirkt die beispielsweise durch äußere oxidative oder reduktive Einflüsse hervorgerufene Oxidationszahländerung störend oder die so veränderten Gläser oder Glaspulver sind für eine weitere Verwendung vollkommen unbrauchbar.

Auf der anderen Seite kann gerade eine gezielte, nachträgliche Änderung der einmal während der Schmelze eingestellten Oxidationszahl einer polyvalenten Glaskomponente erwünscht und sinnvoll sein.

Die Einhaltung einer bestimmten Färbung ist, insbesondere bei Glaspulvern für die Verwendung im Dentalbereich, beispielsweise in einem Dentalglas-Kunststoff-Komposit zur Zahnfüllung, häufig zwingend notwendig. Durch ein Weiterverarbeitungsverfahren oder eine Nachbehandlung des Dental-Glaspulvers hervorgerufene Farbänderungen würden das Glaspulver unbrauchbar machen. Oftmals reichen dabei Spuren von Verunreinigungen der Glaskomponenten aus, um unerwünschte Farbveränderungen während der Weiterverarbeitung eines Dental-Glaspulvers hervorzurufen.

Aufgabe der Erfindung ist es daher, ein Glaspulver bereitzustellen, bei dem sich die Oxidationszahl wenigstens einer Glaskomponente nicht oder nur geringfügig ändert, insbesondere bei einer Weiterverarbeitung oder Nachbehandlung des Glaspulvers durch äußere reduktive Einflüsse. Weiterhin ist es Aufgabe der Erfindung, ein Glaspulver bereitzustellen, bei dem die Oxidationszahl wenigstens einer Glaskomponente nachträglich gezielt eingestellt werden kann. Das Glaspulver soll dabei besonders für die Verwendung im Dentalbereich geeignet sein.

Zur Lösung dieser Aufgabe wird erfindungsgemäß ein Glaspulver, dem wenigstens ein Oxidationsmittel zugesetzt ist, nach Anspruch 1 bereitgestellt, wobei dem Glaspulver NH₄ClO₄ als Oxidationsmittel zugesetzt ist.

Dadurch, daß einem Glaspulver erfindungsgemäß NH₄ClO₄ zugesetzt ist, wird in vorteilhafter und einfacher Weise vermieden, daß sich die Oxidationszahl einer Glaskomponente, insbesondere während äußere reduktive Einflüsse auf das Glaspulver wirken, verändert.

Wirken reduktive Einflüsse, beispielsweise während einer Nachbehandlung, ist dem Glaspulver ein Oxidationsmittel zugesetzt. Art und Menge des Oxidations NH₄ClO₄ mittels werden dabei so gewählt, daß dem jeweiligen äußeren Einfluß gerade entgegengewirkt wird: Bevorzugt ist dem Glaspulver 0,01 bis 1 Gew.-%, insbesondere 0,5 Gew.-% Oxidationsmittel oder Reduktionsmittel zugesetzt.

Die durch äußere Einflüsse hervorgerufene Änderung der Oxidationszahl einer Glaskomponente wird dadurch verhindert oder so gering gehalten, daß der ablaufenden Redoxreaktion entgegengewirkt wird, in dem ein entsprechender Redoxpartner zur Verfügung gestellt wird.

Die Oxidationszahl wenigstens einer Glaskomponente kann aber auch zum Vorteil nachträglich gezielt eingestellt werden. Soll dabei die Oxidationszahl erhöht werden, ist dem Glaspulver wenigstens ein Oxidationsmittel zugesetzt. Auch hierbei kann die Art und Menge des Oxidations mittels einfach experimentell bestimmt werden.

Menge und Art des zugesetzten Oxidations mittels hängen des weiteren insbesondere von der Glaspulverzusammensetzung, der Korngrößenverteilung bzw. der spezifischen Oberfläche, sowie von den jeweiligen äußeren Einflüssen, beispielsweise während der Weiterverarbeitung (z. B. Temperatur; Zeit; Druck; reduktive Atmosphäre) ab.

Findet bei einer thermischen Nachbehandlung eines Glaspulvers eine Reduktion wenigstens einer Glaskomponente statt oder soll die Oxidationszahl wenigstens einer Glaskomponente gezielt erhöht werden, so ist dem Glaspulver vorzugsweise das Oxidationsmittel NH₄ClO₄ zugesetzt. Wobei besonders bevorzugt 0,5 Gew.-% NH₄ClO₄ dem Glaspulver zugesetzt ist.

Dem Glaspulver ist somit wenigstens ein Oxidations mittel zugesetzt, das thermisch aktivierbar ist, d. h. das bei einer Temperaturveränderung, insbesondere einer Temperaturerhöhung seine oxidative Wirkung entfaltet. Dies ist besonders dann von Vorteil, wenn die nachträgliche Änderung des Redoxzustands einer Glaskomponente mit äußeren thermischen Einflüssen einhergeht. Das Oxidations- mittel wird dabei so ausgewählt, daß es in dem Temperaturbereich, in dem das Glaspulver äußeren Einflüssen ausgesetzt ist, seine Wirkung entfaltet.

Weiterhin ist es von Vorteil, dass das Oxidations- mittel nichttoxisch ist. Dies ist neben allgemein ökologischen Anforderungen an ein Glaspulver besonders bei einer Verwendung im Medizin- und Dentalbereich notwendig.

Die Dosierung des Oxidations- mittels kann allerdings i. a. so vorgenommen werden, daß Überschüsse des Oxidationsmittels nach beendeter Reaktion im Glaspulver nicht vorliegen.

Das erfindungsgemäße Glaspulver findet dabei bevorzugt Verwendung als Dentalglaspulver, beispielsweise in einem Dentalglas-Kunststoff-Komposit für die Zahnfüllung.

Das folgende Ausführungsbeispiel soll die Erfindung näher verdeutlichen.

Um die während einer thermischen Nachbehandlung eines Dental-Glaspulvers hervorgerufene Farbveränderung (Nachdunklung) zu vermeiden, wurde dem Glaspulver ein starkes Oxidationsmittel, vorzugsweise etwa 0,5 Gew.-% NH₄ClO₄ zugesetzt. Die ursprüngliche Farbveränderung ließ sich dabei auf sehr geringe Spuren von Titandioxid im Glaspulver zurückführen. Im Temperaturbereich der thermischen Nachbehandlung des Glaspulvers ändert das Titan dabei seinen Redoxzustand irreversibel und bildet stark färbende

Mischoxide. Durch Zusatz von NH₄ClO₄ konnte diese unerwünschte Farbänderung vollständig unterdrückt werden. NH₄ClO₄ entfaltet dabei seine oxidative Wirkung im selben Temperaturbereich, in dem die thermische Nachbehandlung des Dental-Glaspulvers stattfindet.

Ein weiterer Vorteil des NH₄ClO₄-Zusatzes ist, daß die Reaktions- bzw. Zersetzungsprodukte nach der thermischen Nachverarbeitung nichttoxisch sind.

NH₄ClO₄ kann sowohl als Feststoffpulver, als auch in Form einer Lösung dem Glaspulver direkt zugesetzt werden.

Bei der Verwendung von festem NH₄ClO₄ hat es sich als vorteilhaft erwiesen, wenn das NH₄ClO₄ dem Glaspulver während der Mahlung zugesetzt wird, um eine homogene Verteilung zu erreichen. Es ist jedoch auch jede andere Mischmethode denkbar, die eine homogene Verteilung sicherstellt.

Einfacher ist in diesem Zusammenhang das Naßverfahren. Hier wird bei einem Naßverarbeitungsschritt vor der Temperung handelsübliche NH₄ClO₄-Lösung zugegeben. Hier ist darauf zu achten, daß die Homogenisierung im Naßzustand sichergestellt ist. Später wird wie gewohnt getrocknet. Versuche zeigten, daß alle produktionsmäßig eingesetzten Trocknungsverfahren den Oxidationseffekt nicht beeinträchtigten. Fin weiterer Vorteil des Naßverfahrens ist darin zu sehen, daß für NH₄ClO₄-Lösung, im Gegensatz zu festem NH₄ClO₄, keine Lagerbeschränkungen vorliegen.

## Patentansprüche

1. Glaspulver, dem wenigstens ein Oxidationsmittel zugesetzt ist,
**dadurch gekennzeichnet,**
**daß** dem Glaspulver NH₄ClO₄ als Oxidationsmittel zugesetzt ist.

2. Glaspulver nach wenigstens Anspruch 1,
**dadurch gekennzeichnet,**
**daß** dem Glaspulver 0,01 bis 1 Gew.-%, insbesondere 0,5 Gew.-% NH₄ClO₄ zugesetzt ist.

3. Verwendung eines Glaspulvers nach Anspruch 1 oder 2 als Dentalglaspulver.

## Claims

1. Glass powder, to which at least one oxidizing agent is added
**characterized in that**
NH₄ClO₄ is added as oxidizing agent to the glass powder.

2. Glass powder according to Claim 1,
**characterized in that**
0.01 to 1% by weight, in particular 0.5% by weight, of NH₄ClO₄ is added to the glass powder.

3. Use of a glass powder according to Claims 1 or 2 as dental glass powder.

## Revendications

1. Poudre de verre à laquelle est ajouté au moins un agent d'oxydation, **caractérisée en ce que** comme agent d'oxydation, on ajoute à la poudre de verre du NH₄ClO₄.

2. Poudre de verre selon au moins la revendication 1, **caractérisée en ce que** l'on ajoute à la poudre de verre de 0,01 à 1 % en poids de NH₄ClO₄, et en particulier 0,5 % en poids.

3. Utilisation d'une poudre de verre selon la revendication 1 ou 2 comme poudre de verre dentaire.
